# FASCICULE DE BREVET EUROPEEN

(11) **EP 2 303 300 B1**
(45) Date de publication et mention de la délivrance du brevet: **13.03.2013**
(21) Numéro de dépôt: 09793958.1
(22) Date de dépôt: 09.07.2009
(51) Int. Cl.: A61K 36/48, A61K 31/702, A61P 15/02

(54) **COMPOSITIONS VISANT A FAVORISER LE DEVELOPPEMENT ET LA CROISSANCE D'UNE MICROFLORE VAGINALE BENEFIQUE**
ZUSAMMENSETZUNGEN ZUR FÖRDERUNG DER ENTWICKLUNG UND DES WACHSTUMS EINER GÜNSTIGEN VAGINALEN MIKROFLORA
COMPOSITIONS THAT AIM TO PROMOTE THE DEVELOPMENT AND GROWTH OF A BENEFICIAL VAGINAL MICROFLORA

(30) Priorité: 10.07.2008 FR 0854687
(43) Date de publication de la demande: 06.04.2011
(73) Titulaire: Alliospharma, 69380 Lissieu (FR)
(72) Inventeur: BOU ANTOUN, Sami, F-69380 Lissieu (FR)
(74) Mandataire: Regimbeau
(86) Numéro de dépôt international: PCT/EP2009/058756
(87) Numéro de publication internationale: WO 2010/004005

(56) Documents cités:
- EP-A1- 1 348 439
- WO-A1-93/00067
- WO-A1-2005/115170
- WO-A2-02/47493
- WO-A2-2004/084832
- FR-A1- 2 874 825
- DATABASE WPI Section Ch, Week 200876 Thomson Scientific, London, GB; Class B04, AN 2008-M81859 XP002514149 "Compound nutritional albumen powder for preventing osteoporosis of women after menostasis, includes soyabean powder, lactalbumin powder, soybean isoflavone, oligosaccharide, calcium lactate, stabilizer, and lecithin" & CN 101 181 069 A (UNIV TIANJIN SCI & TECHNOLOGY) 21 mai 2008 (2008-05-21)
- DATABASE WPI Week 200320 Thomson Scientific, London, GB; AN 2003-202102 XP002517089 & CN 1 375 492 A (UNIV ZHONGSHAN) 23 octobre 2002 (2002-10-23)
- ROUSSEAU V ET AL: "Prebiotic effects of oligosaccharides on selected vaginal lactobacilli and pathogenic microorganisms" ANAEROBE, LONDON, GB, vol. 11, no. 3, 1 juin 2005 (2005-06-01), pages 145-153, XP004807462 ISSN: 1075-9964
- CHEDRAUI P ET AL: "Red clover extract (MF11RCE) supplementation and postmenopausal vaginal and sexual health" INTERNATIONAL JOURNAL OF GYNECOLOGY AND OBSTETRICS, NEW YORK, NY, US, vol. 95, no. 3, 1 décembre 2006 (2006-12-01), pages 296-297, XP025240894 ISSN: 0020-7292 [extrait le 2006-12-01]

## Description

La présente invention se rapporte à des compositions dermatologiques ou pharmaceutiques visant à favoriser le développement et la croissance d'une microflore vaginale bénéfique.

Chez une femme saine, la flore urogénitale comprend près de 50 espèces différentes de microorganismes. Parmi ces microorganismes, 95% de la population est constituées de diverses souches de lactobacilles. Ces lactobacilles jouent un rôle de protection contre les pathogènes par divers mécanismes : production de peroxyde d'hydrogène, production d'acide lactique, production de bactériocines, inhibition de l'adhésion et de l'expansion des pathogènes. Ainsi, la croissance de nombreux pathogènes de la flore vaginale tels que *Gardnerella vaginalis, Prevotella bivia, Neisseria gonorrhoeae, Candida albicans* est inhibée par les lactobacilles. Les lactobacilles spécifiques du vagin sont notamment *Lactobacillus crispatus, Lactobacillus jensenii* et *Lactobacillus vaginalis.*

La flore vaginale normale est ainsi principalement composée de lactobacilles formant un biofilm protecteur à la surface de la muqueuse. Ces lactobacilles coexistent avec une multitude d'autres espèces dont des pathogènes potentiels. Une rupture de cet équilibre peut être un facteur qui induit des vaginoses dues à une augmentation du pH vaginal et à une diminution des lactobacilles, laissant alors aux pathogènes la possibilité de se développer. Ces déséquilibres se manifestement notamment, mais pas exclusivement, chez les femmes ménopausées.

L'antibiothérapie est l'un des traitements communément utilisé pour traiter les déséquilibres de la microflore vaginale. Il présente cependant les inconvénients habituels des traitements antibiotiques et s'avère de moins en moins efficace. En outre, il vise à éliminer la flore pathogène mais détruit également la flore normale.

Les produits de soin de la muqueuse vaginale déjà existants sur le marché, visant à favoriser le développement et la croissance de la microflore bénéfique du vagin, souffrent d'une efficacité insuffisante.

La présente invention montre de façon inattendue que la combinaison d'un oligosaccharide prébiotique choisi parmi les GOS et les FOS avec un extrait végétal contenant des isoflavones choisi notamment parmi les extraits de soja et de trèfle a un effet synergique sur la croissance des lactobacilles bénéfiques de la flore vaginale. Le GOS en lui-même a un effet prébiotique sur la croissance des lactobacilles. Ce résultat est attendu et a déjà été décrit dans l'état de la technique. De façon surprenante, cet effet prébiotique est cependant amélioré en présence d'un extrait végétal comprenant des isoflavones. Ainsi, l'extrait de trèfle accroît la croissance et la consommation de glucose des lactobacilles. Ce résultat est inattendu car les extraits de végétaux contenant des isoflavones sont connus pour des effets « hormone-like » et sont utilisés pour compenser des déficits en oestrogènes notamment chez les femmes ménopausées. Un effet direct de ces extraits végétaux contenant des isoflavones sur la croissance des lactobacilles n'avait jamais été documenté auparavant.

Le document Database WPI XP-002514149 décrit des compositions comprenant des FOS/GOS et des extraits de soja pour la prévention de l'ostéoporose chez la femme. Ce document ne décrit pas d'utilisation de ces compositions pour le traitement et la prévention des effets de la ménopause sur la muqueuse vaginale ou pour le traitement et la prévention des vaginoses bactériennes. Par ailleurs, ce document ne décrit pas non plus de compositions comprenant des extraits de trèfle.

Le FR 2 874 825 décrit l'utilisation d'oligosaccharides prébiotiques bénéfiques pour la flore vaginale. Des oligosaccharides prébiotiques sont sélectionnés pour leur capacité à favoriser la croissance de souches bactériennes vaginales endogènes bénéfiques. Ce document ne décrit pas de compositions comprenant à la fois des oligosaccharides et des extraits végétaux comprenant des isoflavones.

Le WO93/00067 décrit des compositions cosmétiques comprenant des oligosaccharides. Ces compositions maintiennent un milieu favorable au développement de la flore endogène bénéfique. Des exemples de compositions sont un savon liquide, un shampooing, un lait corporel, une crème pour le visage ainsi qu'un gel vaginal. Le WO93/00067 ne décrit cependant pas de compositions comprenant à la fois des oligosaccharides et des extraits végétaux comprenant des isoflavones.

Le document Rousseau et al. (Anaerobe, 11 :145-153, 2005) décrit les effets prébiotiques de certains oligosaccharides sur les lactobacilles vaginaux et sur des microorganismes pathogènes. Ce document ne décrit pas de compositions comprenant des extraits végétaux contenant des isoflavones.

Le WO2004/084832 décrit des compositions comprenant des extraits de Fo-Ti, de trèfle rouge ou de soja ayant un effet de type oestrogène. Ces compositions conviennent notamment pour le traitement des effets liés à la diminution des oestrogènes lors de la ménopause notamment. Ce document ne décrit cependant pas de compositions comprenant également des GOS et des FOS.

Le EP-A-1 348 439 décrit des compositions pour l'hygiène vaginale comprenant des isoflavones et un extrait de mimosa. Ce document ne décrit pas de compositions comprenant également des oligosaccharides.

Le document Database WPI XP-002517089 décrit une méthode d'extraction d'isoflavones du soja. Ces isoflavones peuvent ensuite être utilisées pour le traitement des bouffées de chaleur et des vaginoses chez les femmes ménopausées, pour la prévention des maladies cardio-vasculaires ainsi que pour la prévention des cancers. Ce document ne décrit pas de compositions comprenant également des oligosaccharides.

L'état de la technique, se rapportant à l'utilisation d'extraits végétaux dans des compositions vaginales, n'enseigne donc pas une amélioration de l'effet prébiotique des oligosaccharides lorsqu'ils sont combinés avec des extraits de trèfle ou de soja.

La présente demande démontre que la combinaison d'un oligosaccharide prébiotique choisi parmi les GOS et les FOS avec un extrait végétal contenant des isoflavones choisi notamment parmi les extraits de soja et de trèfle a un effet prébiotique accru sur les lactobacilles bénéfiques de la flore vaginale.

Ces compositions sont donc particulièrement adaptées au traitement des déséquilibres de la flore vaginale tel que les déséquilibres faisant suite à des vaginoses bactériennes, à des candidoses, à des mycoses. De tels déséquilibres de la flore vaginale peuvent être favorisés par la ménopause.

Les vaginoses, candidoses et mycoses sont habituellement traitées par des antibiotiques. Suite à une cure d'antibiotiques, les compositions de la présente invention permettent de restaurer la microflore vaginale bénéfique et de restaurer le pH physiologique du vagin.

L'invention se rapporte également à des utilisations non thérapeutiques des compositions de la présente invention. Les compositions de la présent invention peuvent être utilisés pour le soin ou l'hygiène vaginale quotidienne afin de maintenir une flore vaginale bénéfique et afin de maintenir le pH physiologique du vagin.

L'invention concerne des compositions comprenant :
- un prébiotique choisi parmi les gluco-oligossaccharides (GOS), les fructo-oligosaccharides (FOS), et leurs mélanges ; et
- un extrait végétal contenant au moins 2, 5, 10, 15, 20, 30, 40, 50 ou 100 ppm d'isoflavones.

L'invention concerne des compositions comprenant :
- un prébiotique choisi parmi les gluco-oligossaccharides (GOS), les fructo-oligosaccharides (FOS), et leurs mélanges ; et
- un extrait végétal contenant au moins 2, 5, 10, 15, 20, 30, 40, 50 ou 100 ppm de formonétine, de biochanine A, de génistéine, de daidzéine, de génistine, de daidzine et/ou d'equol.

L'invention concerne des compositions comprenant :
- un prébiotique choisi parmi les gluco-oligossaccharides (GOS), les fructo-oligosaccharides (FOS), et leurs mélanges ; et
- un extrait végétal contenant au moins 2, 5, 10, 15, 20, 30, 40, 50 ou 100 ppm de daidzéine, de genistéine et/ou de formonetine.

L'invention concerne donc des compositions comprenant :
- un prébiotique choisi parmi les gluco-oligossaccharides (GOS), les fructo-oligosaccharides (FOS), et leurs mélanges ; et
- un extrait végétal contenant des isoflavones choisi parmi les extraits de soja *(Glycine max),* les extraits de trèfle *(Trifolium sp.)* et leurs mélanges.

L'invention concerne plus particulièrement des compositions pour la prévention et le traitement des déséquilibres de la microflore vaginale.

L'invention concerne plus particulièrement des compositions pour la prévention et le traitement des vaginoses bactériennes, des candidoses vaginales et des mycoses vaginales.

De préférence, ces compositions comprennent au moins 0,5%, 1%, 2%, 3%, 4%, 5%, 6% , 7%, 8%, 9% ou 10% de prébiotique et au moins 0,5%, 1%, 2%, 3%, 4%, 5% ou 10 % d'extrait végétal contenant des isoflavones.

De préférence, ces compositions comprennent au moins 0,5% à 7% de prébiotique et 0,5% à 5% d'extrait végétal contenant des isoflavones

Avantageusement, les isoflavones sont choisies parmi la formonétine, la biochanine A, la génistéine, la daidzéine, la génistine,la daidzine, l'equol et leurs mélanges.

Dans un mode de réalisation avantageux, l'extrait végétal est choisi parmi les extraits de soja *(Glycine max),* les extraits de trèfle *(Trifolium sp.)* et leurs mélanges.

De préférence, l'extrait végétal est un extrait de trèfle choisi parmi les variétés *Trifolium subterraneum L., Trifolium repens L* et *Trifoliumi pratense L.*

Plus préférentiellement, l'extrait végétal est un extrait de *Trifolium pratense L* contenant au moins de la génisteine, de la daidzéine et de la formonétine.

Dans un mode de réalisation de l'invention, le prébiotique comprend un GOS α-1,6/α-1,4 ayant un degré de polymérisation d'au moins 4.

Dans un autre mode de réalisation, le prébiotique comprend un FOS comprenant un mélange de FOS ayant un degré de polymérisation de 4, 5 et 6.

La composition peut être sous la forme d'un gel vaginal, d'un savon, d'un spray ou d'une crème pour une application vaginale.

L'invention se rapporte aussi à l'utilisation des compositions pour l'hygiène et le soin vaginal. En particulier, l'invention a pour objet l'utilisation non thérapeutique d'une composition comprenant un prébiotique et un extrait végétal contenant des isoflavones pour l'hygiène et le soin vaginal dans une administration vaginale topique.

Un autre objet de l'invention est l'utilisation non thérapeutique de ces compositions pour maintenir une microflore vaginale bénéfique et /ou pour maintenir le pH vaginal à un pH physiologique de l'ordre de 4.5.

L'invention a donc pour objet des compositions dermatologiques ou pharmaceutiques comprenant :
- un prébiotique choisi parmi les gluco-oligossaccharides (GOS), les fructo-oligosaccharides (FOS), et leurs mélanges ; et
- un extrait végétal contenant des isoflavones.

Les prébiotiques sont généralement des oligosaccharides ou des polysaccharides à courte chaîne constitués approximativement de deux à vingt unités de sucre. Il s'agit de composés non digestibles qui exercent une action bénéfique sur la santé en stimulant sélectivement la croissance et/ou l'activité métabolique d'une ou d'un nombre limité de bactéries. Les prébiotiques sélectionnés dans les compositions de la présente invention agissent comme un substrat sélectif d'une ou d'un nombre restreint de bactéries bénéfiques qui résident dans le vagin et qui stimulent la croissance de celles-ci. Les prébiotiques utilisés dans les compositions de la présente invention ciblent plus particulièrement *Lactobacillus crispatus, Lactobacillus jensenii, Lactobacillus gasseri, Lactobacillus aciduphilus. Lactobacillus iners* et *Lactobacillus vaginalis.*

La plupart des prébiotiques disponibles commercialement sont des oligosaccharides, extraits de matrices végétales naturelles ou obtenus par synthèse (chimique ou biotechnologique). Il existe différents types d'oligosaccharides à potentiel prébiotique tels que les glucooligossacharides (GOS), les fructooligosaccharides (FOS), les galactooligosaccharides, etc. Les oligosaccharides respectivement prébiotiques vis-à-vis de l'intestin et du vagin sont différents. En effet, l'intestin est constitué de nombreuses enzymes qui risquent de dégrader l'oligosaccharide. Celui-ci doit avoir une structure chimique longue et complexe lui permettant de résister à l'attaque enzymatique. A l'inverse, dans le vagin, en l'absence de contraintes enzymatiques, les oligosaccharides prébiotiques possèdent des structures plus courtes et plus simples.

Les FOS sont des fructo-oligosaccharides à courte chaine constitués de molécules de fructose et pouvant contenir une molécule de glucose à l'une des extrémités (en fonction de l'origine du FOS). Les FOS existent à l'état naturel dans un certain nombre de plantes comme l'oignon, la tomate, l'artichaut, la banane, le topinambour, l'asperge, le poireau, le blé, le seigle et l'ail.

Les FOS peuvent être produits industriellement par 2 procédés différents :
Le FOS Raftilose® (Orafti, Belgique) provenant de l'hydrolyse de l'inuline de chicorée. Il existe également d'autres producteurs de ce type de FOS.

Le FOS Actilight® (Béghin Meiji, France) obtenu par synthèse enzymatique. Le FOS Actilight® est produit à partir de saccharose et d'une enzyme, la β-fructosyl transférase selon le mécanisme suivant : 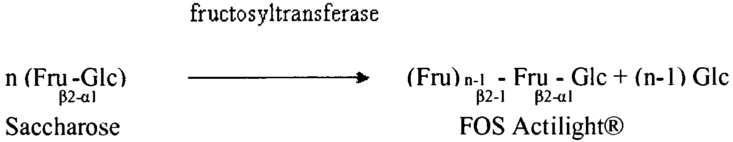 dans laquelle n est de préférence égal à 3, 4 ou 5.

La fructosyl-transferase est produite par *Aspergillus niger* ou *Aureobasidium pullulans.* A partir de n molécules de saccharose, un mélange de saccharose, glucose et de fructooligosaccharides est obtenu. Le FOS Actilight® est ensuite purifié par chromatographie.

Les FOS Actilight® contiennent des degrés de polymérisation allant jusqu'à 6 et dont la proportion de glucose, fructose et saccharose est en moyenne de 5% :
**Structure du FOS Actilight®**

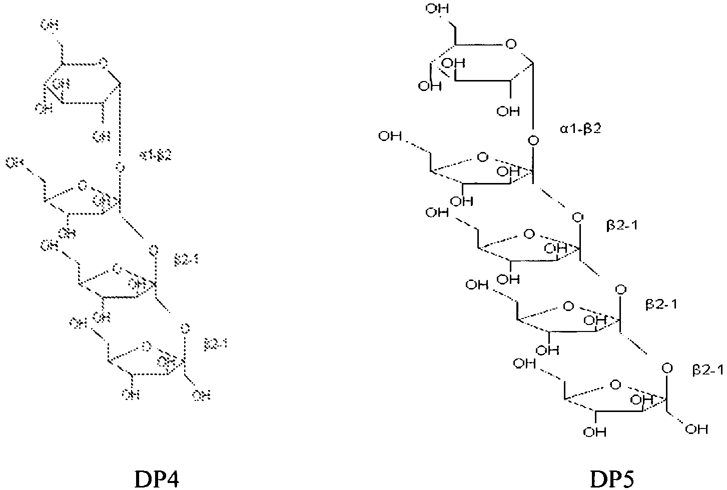

Les GOS sont des gluco-oligosaccharides à courte chaine constitués d'un enchainement d'unités glucose liées en α1-6 et pouvant contenir également des liaisons α1-2, α1-3, α1-4.

Le GOS α1-6 est un gluco-oligosaccharide linéaire constitué d'un enchainement d'unités glucose liées entre elles par des liaisons α1-6. Il est obtenu par une réaction de transglucosylation enzymatique à partir d'un donneur, le saccharose, sur un accepteur qui peut être le glucose, l'alpha-glucoside de méthyle, l'isomaltose, l'isomaltotriose et le maltose. Si l'accepteur est le maltose (glucoses liés en α1-4) alors on aboutit à la synthèse enzymatique de GOSα1-6/α1-4

Les GOS sont des glucanes de petite taille. Ils sont synthétisés par une réaction de transglucosylation catalysée par des enzymes de la famille des glucane-saccharases. Ces enzymes assurent le transfert d'unités glucosyle provenant du substrat donneur sur le produit accepteur à son extrémité non réductrice. L'oligosaccharide ainsi produit devient alors substrat pour donner un oligosaccharide de degré de polymérisation supérieur. Les glucane-saccharases acceptent un nombre limité de substrats donneurs (en général du saccharose) mais un nombre variable de produits accepteurs (par exemple, glucose ou maltose).

Il existe une grande variété de glucane-saccharases qui présentent différents spécificités et catalysent la formation de différents types de liaisons osidiques (α-(1-2), α-(1-3), α-(1-4), α-(1-6)). Les rendements de la réaction dépendent fortement du ratio [donneur]/[accepteur] et de la teneur en sucres totaux dans le milieu. La vitesse de réaction dépend du type d'accepteur; par exemple, la synthèse avec comme accepteur du maltose est plus efficace qu'avec du glucose.

### Synthèse enzymatique du GOSα1-6/α1-4

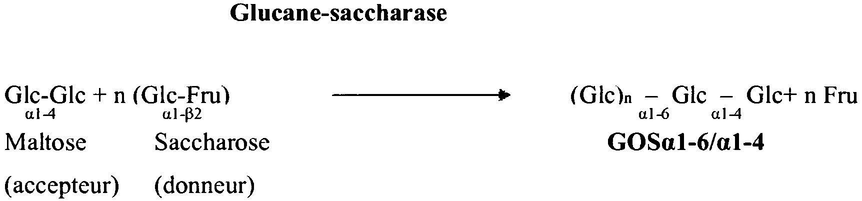

Cette réaction enzymatique est catalysée par la glucane-saccharase de *Leuconostoc mesenteroides* NRRL B-512 (F.)

Les GOSα-1,6/α-1,4 sont des mélanges d'oligosaccharides. Le mélange est constitué d'oligosaccharides allant jusqu'au degré de polymérisation 11.

| GOS | DONNEUR | ACCEPTEUR | ENZYME | LIAISONS |
|---|---|---|---|---|
| **GOSα1-2/α1-6/α1-4** | saccharose | maltose | B-1299 (1) | 65% α1-6 |
| | | | | 35% α1-2 |
| | | | | 5% α1-3 |
| **GOSα1-6/α1-4** | saccharose | maltose | B-512F (2) | 95% α1-6 |
| | | | | 5% α1-35 |

| | | | | |
|---|---|---|---|---|
| (1) glucane-saccharase de *Leuconostoc mesenteroides* NRRL B-1299 (2) glucane-saccharase de *Leuconostoc mesenteroides* NRRL B-512F | | | | |

### Structure du GOSα1-6/α1-4

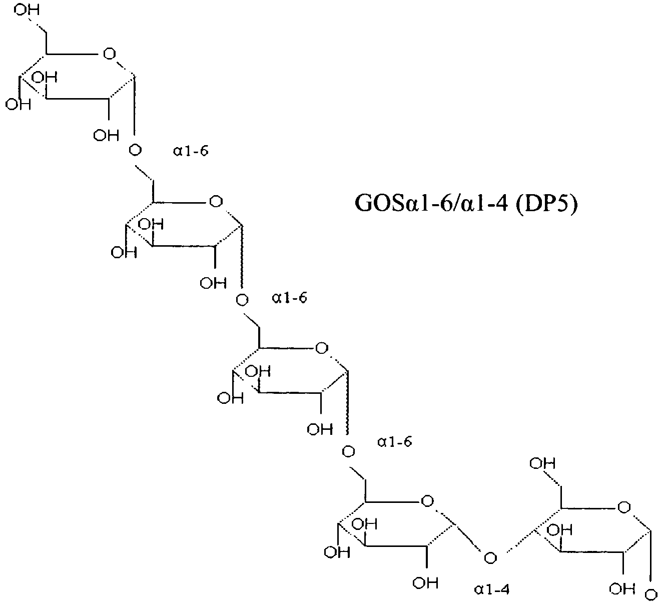

Parmi les prébiotiques utilisables dans les compositions de la présente invention, on citera plus particulièrement les oligosaccharides décrits dans le EP-B-0 591 443 et notamment le GOSα1-2/α1-6 ayant un degré de polymérisation d'au moins 4. Un autre prébiotique préféré est le FOS Actilight® de DP3.

Dans un mode de réalisation préféré de l'invention, les compositions comprennent un mélange de prébiotiques spécifiques de la microflore du vagin.

Les compositions selon la présente invention comprennent également des isoflavones extraites des plantes ayant une structure spatiale similaire aux oestrogènes. Les effets des phytoestrogènes sont connus, ils améliorent la cytologie vaginale, la trophicité et l'hydratation de la muqueuse vaginale ce qui contribue à rendre les conditions de croissance de la microflore du vagin plus favorables. De façon surprenante, ces isoflavones ont également un effet direct sur la croissance de la microflore baginale.

Parmi les nombreuses sources végétales de phytoestrogènes connues, sont particulièrement préférés les extraits de soja *(Glycine max),* les extraits de trèfle *(Trifoliumi sp*.) et leurs mélanges. L'extrait végétal est typiquement sous forme glycériné ou glycolique ou aqueux ou sec ou au CO₂ supercritique.

Ces extraits végétaux comprennent au moins 2, 5, 10, 15, 20, 30, 40, 50 ou 100 ppm d'isoflavones, de préférence de 2 à 20 ppm d'isoflavones.

De préférence, les isoflavones sont choisies parmi la formonétine, la biochanine A, la génistéine, la daidzéine, la génistine, la daidzine, l'equol, et leurs mélanges. Plus préférentiellement, les extraits végétaux comprennent de la daidzéine, de la genistéine et de la formononetine.

Des extraits de végétaux comprenant des isoflavones en quantité suffisante sont préparés selon des techniques bien connues de l'homme du métier. Ces extraits sont préparés avantageusement à partir de trèfle ou de soja. Ces extraits sont disponibles commercialement et on citera à titre d'exemple l'extrait de trèfle Stérocare® de la société Sederma.

Dans un mode de réalisation préféré, les compositions de la présente invention comprennent un extrait de trèfle *Trifolium pratense L* contenant au moins 2 ppm de daidzéine, au moins 11 ppm de génistéine et au moins 9 ppm de formononetine.

Les compositions peuvent en outre comprendre au moins un agent gélifiant d'origine naturelle végétale, ou minérale ou de synthèse, formant au contact de l'eau une gelée de type « hydrocolloïdale » de nature thixotrope, non chargée électriquement. Il peut s'agir de polymères dérivés de l'acide acrylique, d'hydroxyethylcellulose, de carboxymethylcellulose, de gommes xanthane, de guar, de galactomanannes, de succinoglycanes, de carrhagenhannes kappa ou iota, de bentonites, d'hectorites, de silicates de magnésium. L'agent gélifiant représente de 0,5 à 5 % dans la composition finale

Les compositions peuvent également comprendre un glycol qui peut être du monopropylène glycol, du dipropylène glycol, de la glycérine, di-glycérine, triglycérine, du monopropanediol, du butylène glycol, de l'hexylène glycol. Il peut être ajouté à la composition dans la proportion de 1 à 80 % de préférence de 3 à 10% de la composition totale.

Les compositions selon l'invention peuvent aussi comprendre un système conservateur composé de 1 à 4 agents fongistatiques et bactériostatiques, sous forme de sels d'acides organiques : lactique, citrique, sorbique, benzoïque, salicylique, tartrique, glycolique, etc. Ils peuvent être ajoutés à la composition entre 0,1 et 2 % de la composition totale.

Avantageusement, les compositions comprendront des ré-ajusteurs de pH destinés à amener la préparation à pH physiologique : acide citrique, lactique, glycolique, chlorhydrique, borique, hydroxyde de sodium, hydroxyde de potasssium, AMP cyclique, triéthanolamine, trométhamine, ethylène diamine tetraacétique, tampon citrique /citrates, etc. Le ou les ré-ajusteurs sont ajoutés à la composition entre 0,05% et 5% particulièrement entre 0,05% et 1% de la composition finale.

Les compositions peuvent en outre comprendre au moins un composé choisi dans le groupe constitué par :
- des actifs classiquement utilisés en dermatologie tels que les émollients, les actifs hydratants, les agents restructurants de la barrière cutanée, les agents anti-irritants, les agents apaisants,
- des principes actifs ayant une action thérapeutique complémentaire, tels que les antibiotiques, les agents anti-bactériens, les composés antifongiques, les agents anti-viraux, les agents anti-inflammatoires, etc.

Les compositions selon l'invention peuvent être formulées sous la forme de différentes préparations adaptées à une administration topique, à une administration vaginale.

Selon un mode de réalisation préféré, les différentes préparations sont adaptées à l'administration topique et incluent les crèmes, les émulsions, les laits, les pommades, les lotions, les huiles, les solutions aqueuses ou hydro -alcooliques ou glycoliques, les poudres, les sprays, les gelées, les gels, hydrogels ou tout autre produit pour application externe.

L'invention concerne aussi des compositions pharmaceutiques comprenant un prébiotique et un extrait végétal contenant des isoflavones tels que définis ci-dessus ainsi qu'un véhicule pharmaceutique approprié.

De préférence, les compositions pharmaceutiques de la présente invention se présentent sous la forme d'un gel, d'un savon, d'un spray ou d'une crème pour une application topique vaginale.

Les compositions selon l'invention conviennent plus particulièrement pour le traitement et la prévention des déséquilibres de la microflore vaginale et notamment pour la prévention et le traitement des vaginoses bactériennes, des candidoses vaginales et des mycoses vaginales.

Les effets de la ménopause sur les muqueuses peuvent plus particulièrement favoriser le développement de ces déséquilibres de la microflore vaginale.

On entend par vaginose ou vaginose bactérienne un déséquilibre de la flore microbienne du vagin. Elle se caractérise par la disparition des lactobacilles et la multiplication de germes anaérobies tels que le *Gardnerella vaginalis.* Elle témoigne d'un déséquilibre de la flore vaginale avec disparition de l'effet protecteur du bacille de Döderlein.

On entend par déséquilibre de la microflore vaginale tout déficit en lactobacilles et notamment en *Lactobacillus crispatus, Lactobacillus jensenii, Lactobacillus gasseri, Lactobacillus aciduphilus, Lactobacillus iners* et *Lactobacillus vaginalis.*

On entend par candidose vaginale un déséquilibre de la microflore vaginale ou une infection vaginale causée par des levures du genre *Candida.*

On entend par mycoses vaginales des déséquilibres de la microflore vaginale causées plus généralement par des champignons.

L'invention se rapporte aussi à l'utilisation d'une de ces compositions pour la fabrication d'un médicament pour le traitement et la prévention des déséquilibres de la microflore vaginale et plus particulièrement pour le traitement et la prévention des vaginoses bactériennes, des candidoses vaginales et des mycoses vaginales.

L'invention se rapporte aussi à l'utilisation d'un prébiotique et d'un extrait végétal contenant des isoflavones tel que définis ci-dessus pour la fabrication d'un médicament pour le traitement et la prévention des déséquilibres de la microflore vaginale et plus particulièrement pour le traitement et la prévention des vaginoses bactériennes, des candidoses vaginales et des mycoses vaginales.

L'invention concerne aussi des compositions dermatologiques pour l'hygiène vaginale et le soin vaginal. Les compositions selon la présente invention sont particulièrement adaptées pour l'hygiène et le soin vaginal des femmes ménopausées.

Dans le cadre d'une utilisation dermatologique, la composition sera avantageusement formulée sous la forme d'une préparation adaptée à une administration topique.

L'invention a aussi pour objet des méthodes d'hygiène vaginale et de soin vaginal, notamment pour les femmes ménopausées.

L'invention concerne aussi l'utilisation non thérapeutique des compositions selon l'invention pour le soin et l'hygiène vaginale. En particulier, l'invention a pour objet l'utilisation non thérapeutique d'une composition comprenant un prébiotique et un extrait végétal contenant des isoflavones pour l'hygiène et le soin vaginal dans une administration vaginale topique.

Un autre objet de l'invention est l'utilisation non thérapeutique de ces compositions pour maintenir une microflore vaginale bénéfique et /ou pour maintenir le pH vaginal à un pH physiologique de l'ordre de 4.5.

### Figures

Figure 1 : Croissance BLL 2008 *Lactobacillus crispatus*
Figure 2 : Croissance BLL 2005 *Lactobacillus crispatus*

### Exemples

### Exemple 1 : Gelée vaginale

| **MATIERE PREMIERE** | **%** |
|---|---|
| Eau bio-osmosée Aqua | QSP 100 |
| Glycérine végétale codex Glycerin | 5 |
| GOSα Alpha-glucan / oligo saccharide (Génibio®) | 5,0 |
| Stérocare® (Sederma®) Trifolium Pratense extract | 2,0 |
| Natrosol® 250 HHX.Ph Hydroxyethylcellulose | 1,0 |
| Ronkosal® SB Conservateur | 0,7 |
| Purac® HS 90 Lactic acid | QSP Ph 4, 3 +/- 0,1 0.22 |

La concentration de stérocare peut également varier de 0.5 à 3 %. Le reste de la formule restant inchangé. Pour une formulation en gel, des agents gras sont ajoutés.

Le Stérocare® comprend 2 ppm de daidzéine, 11 ppm de genistéine et 9 ppm de formononetine.

### Exemple 2 : Formule gel placebo

| **MATIERE PREMIERE** | **%** |
|---|---|
| Eau bio-osmosée Aqua | QSP 100 |
| Glycérine végétale codex Glycerin | 5 |
| Natrosol® 250 HHX.Ph Hydroxyethylcellulose | 1,0 |
| Ronkosal® SB Conservateur | 0,7 |
| Purac® HS 90 Lactic acid | QSP Ph 4, 3 +/- 0,2 0.3 |

Il s'agit de la même formule sans le GOS et le stérocare.

### Exemple 3 : Mousse lavante (ou gel d'hygiène intime)

| **MATIERE PREMIERE** | **%** |
|---|---|
| Eau purifiée Aqua | QSP 100 |
| Protéol® APL Sodium Cocoyl apple amino acides Moussant dérivé de la pomme | 15 |
| Laureth P065 Aqua, coco-glucoside, glyceryl oleate | 3 |
| GOSα Alpha-glucan / oligo saccharide (Génibio®) | 1 |
| Trèfle rouge HG Trifolium Pratense, glycerine, eau Actif (Greentech®) | 1 |
| | |
| Sepinov® EMT10 Hydroxy ethyl acrylate/sodium (agent stabilisant) | 0.7 |
| Suttocide® A Sodium methyl hydroxy glycinate (conservateur) | 0.5 |
| Purac®) HS 90 acide lactique Ajusteur de PH | 0.4 (PH 5-5.5) |
| Thé vert menthe 12309 (parfum) | 0.06 |
| Colorant rouge autorisé CE CI 16035 | 0.00066 |
| EDTA Ethylene diamine tetra acétique (agent séquestrant) | 0.05 |

Les concentrations en GOS et en trèfle rouge peuvent varier entre 0,5 et 1,5%.

### Exemple 4 : Gélule

Oligosaccharides prébiotiques (GOS et/ou FOS)
Isoflavones (de trèfle et/ou de soja)
Excipients : lactose monohydraté, amidon de pomme de terre, glutamate de sodium, thiosulfate de sodium, enveloppe de la gélule (dioxyde de titane E171, gélatine).

### Exemple 5 : Crème

Oligosaccharides prébiotiques (GOS et/ou FOS)
Isoflavones (de trèfle et/ou de soja)
Excipients : vaseline, cire émulsionnable non ionique au cétamacrogol 1000, paraffine liquide, glycine, acide sorbique, solution concentrée d'hydroxyde de sodium qsp PH 4-5, eau purifiée.

### Exemple 6 :

Nous avons choisi deux souches de Lactobacilles BLL 2005 et 2008 (les deux souches les plus significatives en terme d'activité probiotique) pour lesquelles nous avons testé d'une part, le glucose seul qui le substrat universel des Lactobacilles, et le glucose + Stérocare (1,5% et 3%), et d'autre part, le GOS seul et le GOS + Stérocare (1,5% et 3%).

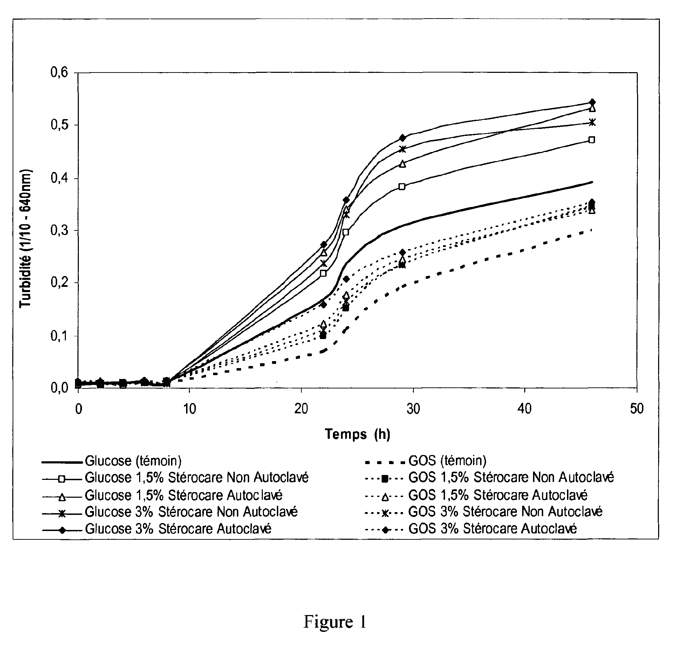

Dans les deux cas, nous observons que le Stérocare augmente la consommation de substrats par les Lactobacilles et donc leur croissance (Figure 1).

### Exemple 7 : Suivi de la croissance de bactéries lactiques : croissance en présence ou non de gel hygiène vaginale

Concentration initiale en sucres 5g/l, et inoculation à 2%

Les gels d'hygiène vaginale formule active et formule placébo des exemples 1 et 2 ont été utilisés.

| | **BLL *2005 - Lactobacillus crispatus*** | | | | | |
|---|---|---|---|---|---|---|
| **Temps (h)** | **MRS + Glucose** | | **MRS + Placebo** | | **MRS + Gel actif** | |
| | **Turbidité 1/10 - 640 nm** | **pH** | **Turbidité 1/10 - 640 nm** | **pH** | **Turbidité 1/10 - 640 nm** | **pH** |
| **Pré-culture** | 0,328 | 4,0 | | / | / | / |
| 0,0 | 0,005 | 6,5 | 0,004 | 6,4 | 0,005 | 6,4 |
| 2,5 | 0,005 | / | 0,005 | / | 0,004 | / |
| 4,0 | 0,008 | / | 0,007 | / | 0,005 | / |
| 6,0 | 0,014 | 4,9 | 0,011 | 6,0 | 0,011 | 6,2 |
| 24,0 | 0,214 | 4,9 | 0,022 | 6,0 | 0,166 | 5,2 |
| 29,5 | 0,224 | / | 0,022 | / | 0,190 | / |
| 46,5 | 0,195 | 4,9 | 0,021 | 6,0 | 0,185 | 5,1 |

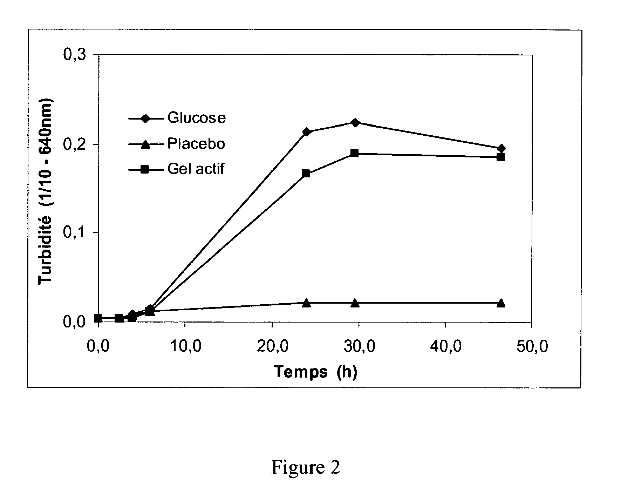

Les résultats ci-dessus et la figure 2 montrent sans ambiguïté que le gel actif permet la croissance de la souche alors que ce n'est pas le cas avec le placébo.

### Exemple 8 : Comparaison des scores de Nugent et du pH vaginal chez des femmes traitées et non traitées

### Protocole

Traitement de deux groupes de femmes ayant eu une vaginose bactérienne et traitées par un antibiotique. Le premier groupe reçoit le produit actif et le second (placebo) reçoit le produit sans les prébiotiques et l'extrait de trèfle.

Le traitement consiste à appliquer une fois par jour les produits (celui à base de prébiotiques et isoflavones et le placebo). Le produit se présente sous forme de tubes monodoses à usage unique contenant une le GOS à 5% et l'extrait végétal contenant les isoflavones à 2% (gelée vaginale exemple 1).

Le traitement est quotidien pendant 8 jours.

A J0, les femmes sont auscultées par un gynécologue qui effectue un prélèvement vaginal et mesure le pH vaginal.

A J8, une deuxième visite et prélèvement vaginal et mesure du pH.

Il s'agit de mesurer le score de Nugent à J0 et J8 ainsi que le pH, comparer les scores et les pH au sein du même groupe et en comparant les sujets des deux groupes.

### Analyse des scores de Nugent

Objectif du score de Nugent :
- Déterminer la qualité de la flore vaginale
- Différencier une flore vaginale normale, intermédiaire (pouvant évoluer vers une vaginose) et les vaginoses.

Comparaison de l'évolution des scores de Nugent entre le groupe actif et placebo

| **Sujets** | **Score de Nugent** | | | | | |
|---|---|---|---|---|---|---|
| | **Zone normale** | | **Zone intermédiaire** | | **Vaginose** | |
| | **(0-3)** | | **(4-6)** | | **(7-10)** | |
| | **D0** | **D8** | **D0** | **D8** | **D0** | **D8** |
| **Actifs** | **72%** | **100%** | **28%** | **0%** | **0%** | **0%** |
| **Placebos** | **67%** | **56%** | **33%** | **33%** | **0%** | **11%** |

### ➢ Au bout de 8 jours :

Le Groupe actif montre au bout de 8 jours de traitement une restauration d'une flore vaginale normale pour tous les sujets.

Le groupe placebo montre une dispersion plus aléatoire au bout de 8 jours avec 33% d'individus caractérisées par une flore intermédiaire et 11 % par une vaginose.

Analyse statistique des scores de Nugent à D8.

| **Score de Nugent D8** | **Moyenne** | **Ecart type** | **Intervalle** |
|---|---|---|---|
| **Actives (8)** | **1** | **1** | **[0-2]** |
| **Placebos (9)** | **3** | **3,67** | **[0-6,6]** |

Une plus grande variabilité des scores de Nugent est observée chez le groupe placebo vs. le groupe actif. L'écart type est en effet 2.5 fois plus important dans la population placebo.

Evolution cinétique des scores de Nugent du groupe actif au bout de 8 jours

| | **Stabilisation d'une flore vaginale normale** | **Restauration d'une flore vaginale normale** |
|---|---|---|
| **Actifs (8)** | 75% | 25% |

Le traitement conduit à un score de Nugent qui se stabilise en deçà de 3 au bout de 8 jours.
75% des sujets ont un score <1
12.5 % des sujets ont un score =2
12,5 % des individus ont un score passant de 5 à 3.

### Conclusion :

Sur la base de cet échantillon, le traitement a un effet significatif sur le groupe actif et conduit à rétablir plus rapidement une flore vaginale normale.

11 % des individus placebos refont une vaginose au bout de 8 jours.

### Analyse du pH vaginal

Le pH physiologique de la muquesue vaginale est acide et de l'ordre de 4.5.

Une vaginose est caractérisée par un pH supérieur.

Comparaison de l'évolution du pH vaginal entre le groupe actif et placebo

| **Sujets** | **pH≤4** | | **pH>4** | |
|---|---|---|---|---|
| | **D0** | **D8** | **D0** | **D8** |
| **Actifs** | **50%** | **71%** | **50%** | **29%** |
| **placebos** | **55%** | **33%** | **45%** | **67%** |

### ➢ Au bout de 8 jours :

Le pH vaginal des sujets actifs tend à diminuer au bout de 8 jours de traitement vs. les sujets placebos où on note un effet inverse.

Variation du pH vaginal chez les sujets actifs entre D0 et D8.

| | **A1** | **A3** | **A4** | **A5** | **A7** | **A8** | **A9** |
|---|---|---|---|---|---|---|---|
| Δ**pH (D0-D8)** | **0** | **-1** | **0.5** | **0** | **2** | **2** | **0** |

Variation du pH vaginal chez les sujets placebos entre D0 et D8.

| | **P1** | **P2** | **P3** | **P4** | **P5** | **P6** | **P7** | **P8** | **P9** |
|---|---|---|---|---|---|---|---|---|---|
| Δ**pH (D0-D8)** | **-2** | **0** | **1.5** | **-1** | **-0.5** | **0** | **0** | **0** | **0** |

86 % des sujets actifs montrent soit une stabilisation ou une diminution du pH vaginal.

28% ont un pH final égal à 5.

66 % des sujets placebos montrent soit une stabilisation ou une diminution du pH vaginal, sachant que dans 66% des cas ce pH final est supérieur ou égale à 4.5.

### Conclusion :

Sur la base de cet échantillon, le traitement a un effet significatif sur le groupe actif et conduit :
- A favoriser une diminution du pH pour tendre vers des valeurs physiologiques (4-4.5) Ou,
- A maintenir un pH vaginal physiologique restauré par le traitement antibiotique.

La stabilité ou la diminution du pH vaginal chez les sujets placébos n'atteint une valeur physiologique que dans 33% des cas.
➢ L'effet du traitement sur les sujets actifs à un effet significatif positif sur la qualité de la flore et du pH vaginal.
➢ On note une corrélation globale entre la tendance à une flore normale et la tendance à un pH acide reflétant une prédominance des bactéries lactiques « commensales ».

### Exemple 9 : Dosage des isoflavones

L'objectif de cette étude était de tester la présence de prébiotiques et d'isoflavones dans le sang de lapines suite à l'administration topique d'une composition selon l'invention.

Deux lapines par groupe ont reçu une fois par jour pendant 8 jours une application topique intra-vaginale de gel contrôle (vaseline) et de gel selon l'exemple 1 (Gel contenant le GOS à 5% et l'extrait végétal contenant les isoflavones à 2%) Des échantillons de sang ont été prélevés un jour avant les applications T₀ et un jour après la fin des applications T_{8.}

Par HPLC, il n'a pas été possible de détecter la présence d'isoflavones (diadzéine, genistéine et formononetine) ou de GOS dans le plasma des lapines du groupe traité et du groupe contrôle. A titre de contrôle positif, l'extrait végétal de trèfle utilisé dans les compositions comprend 2 ppm de diadzéine, 11 ppm de genistéine et 9 ppm de formononetine.

Les compositions ont donc uniquement un effet topique, les composants actifs, les GOS et les isoflavones, ne sont pas absorbés et n'ont pas d'effet systémique. Les effets des isoflavones n'est donc pas un effet *« hormone-like* ».

## Revendications

1. Composition pour utilisation dans une administration topique dans la prévention et le traitement des déséquilibres de la microflore vaginale **caractérisée en ce qu'**elle comprend :
- un prébiotique choisi parmi les gluco-oligossaccharides (GOS), les fructo-oligosaccharides (FOS), et leurs mélanges ; et
- un extrait végétal contenant des isoflavones choisies parmi la formonétine, la biochanine A, la génistéine, la daidzéine, la génistine, la daidzine, l'équol et leurs mélanges.

2. Composition selon la revendication 1 pour utilisation dans la prévention et le traitement des vaginoses bactériennes, des candidoses vaginales et des mycoses vaginales.

3. Composition selon la revendication 1 pour utilisation dans la restauration d'une flore vaginale bénéfique suite à un traitement antibiotique.

4. Composition selon la revendication 1 pour utilisation dans la restauration d'un pH vaginal physiologique de l'ordre de 4.5.

5. Composition selon la revendication 1 pour utilisation dans l'hygiène et le soin vaginal dans une administration vaginale topique.

6. Composition selon la revendication 1 pour utilisation dans le maintien d'une microflore vaginale bénéfique.

7. Composition selon la revendication 1 pour utilisation dans le maintien du pH vaginal à un pH physiologique de l'ordre de 4.5.

8. Composition selon l'une des revendications 1-7 **caractérisée en ce qu'**elle comprend 0,5% à 7% de prébiotique et 0,5% à 5% d'extrait végétal contenant des isoflavones.

9. Composition selon l'une des revendications précédentes **caractérisée en ce que** l'extrait végétal comprend au moins 10 ppm d'isoflavones.

10. Composition selon l'une des revendications précédentes **caractérisée en ce que** l'extrait végétal est choisi parmi les extraits de soja *(Glycine max),* les extraits de trèfle *(Trifolium sp.)* et leurs mélanges.

11. Composition selon l'une des revendications précédentes **caractérisée en ce que** l'extrait végétal est un extrait de trèfle choisi parmi les variétés *Trifolium subterraneum L., Trifolium repens L* et *Trifolium pratense L.*

12. Composition selon l'une des revendications précédentes **caractérisée en ce que** l'extrait végétal est un extrait de *Trifolium pratense L* contenant au moins de la génisteine, de la daidzéine et de la formonétine.

13. Composition selon l'une des revendications précédentes caractérisée que le prébiotique comprend un GOS α-1,6/α-1,4 ayant un degré de polymérisation d'au moins 4.

14. Composition selon l'une des revendications précédentes caractérisée que le prébiotique comprend un FOS comprenant un mélange de FOS ayant un degré de polymérisation de 4, 5 et 6.

15. Composition selon l'une des revendications précédentes **caractérisée en ce que** la composition est sous la forme d'un gel vaginal, d'un savon, d'un spray ou d'une crème pour une application vaginale.

## Claims

1. A composition for use in a topical administration for the prevention and treatment of imbalances of the vaginal microflora wherein it contains:
- a prebiotic chosen among gluco-oligosaccharides (GOS), fructo-oligosaccharides (FOS), and mixtures thereof; and
- a plant extract containing isoflavones chosen among formononetin, biochanin A, genistein, daidzein, genistine, daidzine, equol and mixtures thereof.

2. The composition of claim 1 for use for the prevention and treatment of bacterial vaginoses, vaginal candidiases and vaginal mycoses.

3. The composition of claim 1 for use for the restoration of a beneficial vaginal flora following an antibiotic treatment.

4. The composition of claim 1 for use for the restoration of a vaginal physiological pH of roughly 4.5.

5. The composition of claim 1 for use for vaginal hygiene and care in a topical vaginal administration.

6. The composition of claim 1 for use to maintain a beneficial vaginal microflora.

7. The composition of claim 1 for use to maintain a vaginal physiological pH of roughly 4.5.

8. The composition according to one of claims 1 to 7, wherein it contains 0.5% to 7% prebiotic and 0.5% to 5% plant extract containing isoflavones.

9. The composition according to one of the preceding claims, wherein the plant extract contains at least 10 ppm isoflavones.

10. The composition according to one of the preceding claims, wherein the plant extract is chosen among soybean *(Glycine max)* extracts, clover (*Trifolium* sp.) extracts and mixtures thereof.

11. The composition according to one of the preceding claims, wherein the plant extract is a clover extract chosen among the varieties *Trifolium subterraneum L., Trifolium repens* L. and *Trifolium pratense* L.

12. The composition according to one of the preceding claims, wherein the plant extract is an extract of *Trifolium pratense* L. containing at least genistein, daidzein and formononetin.

13. The composition according to one of the preceding claims, wherein the prebiotic comprises an α-1,6/α-1,4 GOS having a degree of polymerization of at least 4.

14. The composition according to one of the preceding claims, wherein the prebiotic comprises a FOS comprised of a mixture of FOS having a degree of polymerization of 4, 5 and 6.

15. The composition according to one of the preceding claims, wherein the composition is in the form of a vaginal gel, a soap, a spray or a cream for vaginal application.

## Patentansprüche

1. Zusammensetzung für die Verwendung in einer topischen Verabreichung bei der Vorbeugung und Behandlung von Ungleichgewichten der vaginalen Mikroflora, **dadurch gekennzeichnet, dass** die Zusammensetzung umfasst:
- ein Präbiotikum, das ausgewählt ist aus Gluco-oligosacchariden (GOS), Fructo-oligosacchariden (FOS) und deren Mischungen; und
- einen Pflanzenextrakt, der Isoflavone enthält, die ausgewählt sind aus Formonetin, Biochanin A, Genistein, Daidzein, Genistin, Daidzin, Equol und deren Mischungen.

2. Zusammensetzung nach Anspruch 1 für die Verwendung bei der Vorbeugung und Behandlung von bakteriellen Vaginosen, vaginalen Kandidosen und vaginalen Mycosen.

3. Zusammensetzung nach Anspruch 1 für die Verwendung bei der Wiederherstellung einer günstigen Vaginalflora nach einer antibiotischen Behandlung.

4. Zusammensetzung nach Anspruch 1 für die Verwendung bei der Wiederherstellung eines physiologischen vaginalen pH-Wertes im Bereich von 4,5.

5. Zusammensetzung nach Anspruch 1 für die Verwendung bei der vaginalen Hygiene und Pflege bei einer topischen vaginalen Verabreichung.

6. Zusammensetzung nach Anspruch 1 für die Verwendung beim Erhalt einer günstigen vaginalen Mikroflora.

7. Zusammensetzung nach Anspruch 1 für die Verwendung bei der Aufrechterhaltung des vaginalen pH-Wertes bei einem physiologischen pH-Wert im Bereich von 4,5.

8. Zusammensetzung nach einem der Ansprüche 1 bis 7, **dadurch gekennzeichnet, dass** sie 0,5 bis 7% Präbiotikum und 0,5% bis 5% Isoflavone enthaltenden Pflanzenextrakt umfasst.

9. Zusammensetzung nach einem der vorangehenden Ansprüche, **dadurch gekennzeichnet, dass** der Pflanzenextrakt mindestens 10 ppm Isoflavone enthält.

10. Zusammensetzung nach einem der vorangehenden Ansprüche, **dadurch gekennzeichnet, dass** der Pflanzenextrakt ausgewählt ist aus Sojaextrakten *(Glycine max),* Kleeextrakten *(Trifolium sp.)* und deren Mischungen.

11. Zusammensetzung nach einem der vorangehenden Ansprüche, **dadurch gekennzeichnet, dass** der Pflanzenextrakt ein Kleeextrakt ist, der ausgewählt ist aus den Varietäten *Trifolium subterraneum L., Trifolium repens L.* und *Trifolium pratense L.*

12. Zusammensetzung nach einem der vorangehenden Ansprüche, **dadurch gekennzeichnet, dass** der Pflanzenextrakt ein Extrakt aus *Trifolium pratense L.* ist, der mindestens Genistein, Daidzein und Formonetin enthält.

13. Zusammensetzung nach einem der vorangehenden Ansprüche, **dadurch gekennzeichnet, dass** das Präbiotikum ein α-1,6/α-1,4-GOS mit einem Polymeristationsgrad von mindestens 4 umfasst.

14. Zusammensetzung nach einem vorangehenden Ansprüche, **dadurch gekennzeichnet, dass** das Präbiotikum ein FOS umfasst, welches eine Mischung von FOS mit einem Polymerisationsgrad von 4, 5 und 6 umfasst.

15. Zusammensetzung nach einem der vorangehenden Ansprüche, **dadurch gekennzeichnet, dass** sie in Form eines Vaginalgels, einer Seife, eines Sprays oder einer Creme für eine vaginale Applikation vorliegt.
